# EUROPEAN PATENT APPLICATION

(11) **EP 4 516 251 A1**
(43) Date of publication of application: **05.03.2025**
(21) Application number: 24177353.0
(22) Date of filing: 22.05.2024
(51) Int. Cl.: A61B 18/14, A61B 18/00

(54) **ELECTRODE FOR HANDHELD ELECTROSURGICAL INSTRUMENT**

(30) Priority: 29.08.2023 US 202363535101 P
(71) Applicant: Olympus Winter & Ibe GmbH, 22045 Hamburg (DE)
(72) Inventor: Hanke, Harald, 22145 Hamburg (DE)
(74) Representative: Hoener, Matthias

(57) **Abstract**

The invention provides an electrode with which electrosurgical treatment can be performed in a reliable and safe manner even in the presence of metallic elements in the area to be treated. For this purpose, it is provided that an electrode (16) for an electrosurgical handheld instrument consists of an electrically conductive wire (18), the two ends of the wire (18) being connectable to an electrode support (14) of the hand instrument. In this case, the wire (18) has two sections R and L, which adjoin the two ends of the electrode (16) and can be aligned parallel to one another and straight. These two sections R and L are adjoined by a section C which connects the two sections R and L to one another. According to the invention, it is provided that the wire (18) has a cross-section (25) of 0.5 mm to 1.0 mm.

## Description

The invention relates to an electrode for an electrosurgical handheld instrument according to claim 1.

Electrosurgical handheld devices, in particular resectoscopes, of the type described are used primarily in urology for electrosurgical work. In this context, these devices are usually used for resection and for evaporation of tissue, for example of tissue in the lower urinary tract. For this purpose, the hand-held device, in particular the resectoscope, may comprise a longitudinally displaceable electrode support which, after insertion of the device into the body to be treated, may be advanced with a distal working end from a distal end of the instrument shaft of the handheld device. An electrosurgical electrode is disposed on the electrode support at a distal end thereof. This electrode may be in the form of a loop, for example, and is pulled or pushed through the tissue to manipulate the tissue, depending on the design of the instrument.

Known electrodes are formed from a wire. For loop electrodes, for example, it is known that the wire is formed into a loop and the two free ends are connected to the electrode support. When the high-frequency alternating voltage is applied, a plasma is ignited at the electrode, so that a plasma is formed on the wire in interaction with the liquid surrounding the electrode. The plasma ignites preferentially at positions of the electrode that have a small radius of curvature, since the electric field strength is greatest there.

In high-frequency surgery, situations have proven problematic in which metallic implants or other elements made of an electrically conductive material have been inserted into the patient in the area of the tissue to be treated during previous treatments. When a high-frequency electrical potential is applied to the electrode, a discharge or spark can occur between the electrode and the metallic element. This leads to an uncontrolled and even unwanted manipulation of the human tissue. The resulting traumatization of the patient cannot be foreseen and should therefore be avoided. Secondly, the electrode experiences a greatly increased degree of erosion due to this discharge, which increases the risk of fracture. In addition, the electrode can be directly melted by a very high current that occurs locally for a short time. The two breaking points of the electrode or the two free ends can lead to serious injury to the patient. In addition, there is a risk of an electric arc forming between the two fracture ends, which in turn leads to uncontrolled manipulation of the tissue.

The invention is therefore based on the problem of creating an electrode with which electrosurgical treatment can be carried out in a reliable and safe manner even in the presence of metallic elements in the area to be treated.

A solution to this problem is described by claim 1. Accordingly, it is provided that an electrode for an electrosurgical handheld instrument, in particular for a resectoscope, consists of an electrically conductive wire, wherein the two ends of the wire can be connected to an electrode support of the handheld instrument. In this case, the wire has two sections R and L, which adjoin the two ends of the electrode support and can be aligned parallel to one another and straight. These two sections R and L are adjoined by a section C which connects the two sections R and L to one another. According to the invention, it is provided that the wire has a cross-section of 0.5 mm to 1.0 mm. This significantly increased cross-section of the electrode wire results in the electrode having sufficient strength even in the event of an unpredictable sparkover and does not break at least during the activated time. Because of the greater mass compared to known electrodes, material loss due to erosion is less serious. A particularly preferred embodiment of the invention may provide that the wire has a cross-section of 0.8 mm.

Preferably, it may be provided that the two sections R and L of the wire are aligned parallel to each other. Equally, however, it is also conceivable that the two sections R and L enclose an angle and/or have a radius. Similarly, it is conceivable that the section C is formed as an arc or loop. Depending on the type of treatment as well as the embodiment of the electrode, it can have almost any shape. For example, it is also conceivable that the sections R, L and C consist of further subsections which enclose an angle or radius with respect to one another. Due to this flexibility in the shape of the electrode, it is conceivable to use a specially formed electrode for each application.

Another particularly advantageous embodiment provides for the wire to be made of an electrically conductive material with a very high melting point, such as tungsten, molybdenum or tantalum. It has been shown that these materials are particularly well suited for the application described above. The melting point should be at least 2000°C, preferably more than 3000°C, in particular more than 3400°C.

Furthermore, it can be provided according to the invention that the wire has a round or a circular cross-section. This choice of shape for the electrode permits particularly cost-effective manufacture. Ultimately, a wire of the above-mentioned material must be bent into the correct shape for the manufacture of the electrode. On the one hand, this production can be carried out without great effort and, above all, it is reproducible, so that the required cost-effective production is guaranteed, especially for so-called *single-use* tools.

Preferably, it may be provided that the sections R, L and C lie in one plane or that the sections R, L and C lie in two different planes. Another embodiment may provide that the sections R, L and C are arranged at a right angle to each other.

A preferred embodiment of the invention is described in more detail below with reference to the drawing. In this show:
- Fig. 1: a schematic representation of a handheld surgical instrument, in particular a resectoscope,
- Fig. 2: a side view of an electrode, and
- Fig. 3: a front view of the electrode.

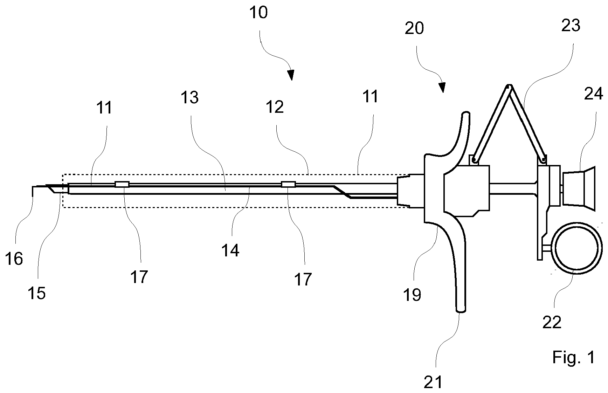

Fig. 1 shows a schematic, side cross-sectional view of a resectoscope 10. The resectoscope 10 has a resectoscope shaft 11 that includes an outer shaft 12 or sheath tube. A tubular inner shaft 13 extends within the outer shaft 12. An electrode support 14 and an indicated optic 15 are shown within the inner shaft 13. In addition, other elements not shown here may be disposed within the resectoscope 10, such as a separate irrigation tube and the like.

The electrode support 14 has an electrosurgical tool or electrode 16 at a distal end. The electrode 16 shown here is shown as a loop, but may have another shape.

The electrode support 14 can be forcibly moved axially in the distal and proximal direction by actuating a handle 19. In doing so, it can be pushed beyond the distal end of the inner shaft 13 and the outer shaft 12. This allows the surgeon to manipulate tissue further away from the resectoscope tip. Furthermore, for this purpose, the inner shaft 13 and/or the electrode support 14 can be rotatably mounted about their longitudinal axis. For the manipulation of the tissue, a high-frequency electric current is applied to the electrode 16.

The resectoscope 10 shown in Fig. 1 has a passive transporter in which a carrier 20 is displaced in the distal direction against the distal, first handle part 21 by relative movement of the handle parts 21 and 22 arranged proximally on the resectoscope shaft 11 against a spring force applied by a spring bridge 23. When the carrier 20 is displaced in the distal direction against the handle part 21, the electrode support 14 is displaced in the distal direction in a manner not shown. When the load on the handle parts 21, 22 is relieved, the spring force generated by the spring bridge 23 forces the carrier 20 back to its initial position, pulling the electrode support 14 in the proximal direction. When the carrier 20 is moved back, an electrosurgical procedure can be performed with the electrode 16 without the manual force of the operator, i.e. passively.

For targeted treatment by means of the electrode 16, the optics 15 are positioned in such a way that the surgeon has an optimal view of the area of the operation. For this purpose, the resectoscope 10 has an ocular 24 at a proximal end, which is connected to the optics 15. Alternatively, it is also conceivable that a camera is arranged on the resectoscope 10 instead of the ocular 24.

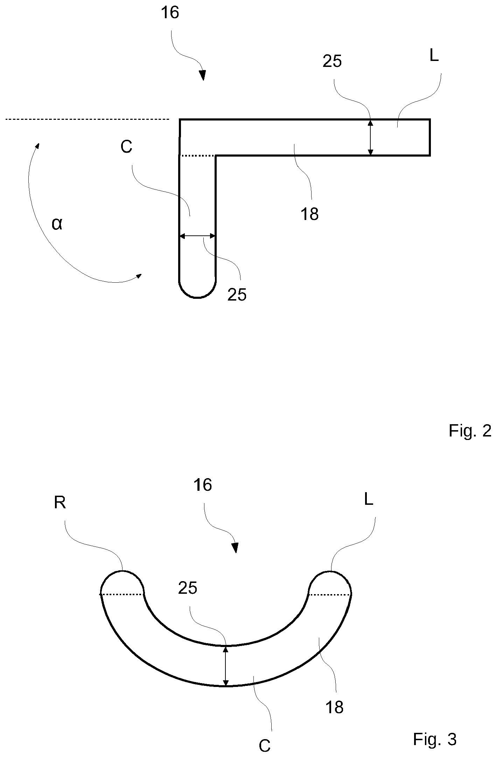

Figs. 2 and 3 show an example embodiment of an electrode 16 according to the invention. This electrode 16 has sections R and L, wherein these sections R and L are pronounced to be of equal length and oriented parallel to each other. Sections R and L are connected to the distal ends of the electrode support tubes.

The central section C is located between the sections R and L. In the embodiment example of the electrode 16 shown here, the section C or the plane in which the section C is located is rotated by an angle of α = 90° with respect to a plane defined by the two sections L and R, respectively. Similarly, it is conceivable that the angle α takes a different value.

The length of the two sections R and L may be a few millimeters up to 2 cm, with a distance between the two sections R and L being a few millimeters. According to the invention described herein, the cross-section 25 of the electrode 16 at all sections R, L and C is 0.5 mm to 1.0 mm, preferably 0.7 mm to 0.9 mm, in particular 0.8 mm. It has been shown that such a dimensioning of the cross-section 25 behaves advantageously, in particular, if further metallic objects are located in the vicinity of the area to be treated.

In addition to the loop shape of the electrode 16 shown here as an example, it is also conceivable that the section C has another shape, such as a loop with a smaller or a larger radius of curvature. Similarly, it is conceivable that the section C comprises a plurality of subsections, each enclosing an angle.

### List of Reference Numerals:

- 10: Resectoscope
- 11: Resectoscope shaft
- 12: Outer shaft
- 13: Inner shaft
- 14: Electrode support
- 15: Optics
- 16: Electrode
- 17: Guide element
- 18: Wire
- 19: Handle
- 20: Carrier
- 21: Handle part
- 22: Handle part
- 23: Spring bridge
- 24: Ocular
- 25: Cross section

- R: Section
- L: Section
- C: Section
- α: Angle

## Claims

1. Electrode (16) for an electrosurgical handheld instrument, in particular for a resectoscope (10), consisting of an electrically conductive wire (18), wherein the two ends of the wire (18) can be connected to an electrode support (14) of the handheld instrument, **characterized in that** the wire (18) has a cross-section (25) of 0.5 mm to 1.0 mm.

2. Electrode (16) for a handheld electrosurgical instrument according to claim 1, **characterized in that** the wire (18) comprises two sections R and L, the two sections R and L having two first ends adjacent to the electrode support (14) and a section C joining the two sections R and L between second ends of the sections R and L.

3. Electrode (16) for an electrosurgical handheld instrument according to claim 1 or 2, **characterized in that** the two sections R and L of the wire (18) are aligned parallel to each other as well as straight.

4. Electrode (16) for an electrosurgical handheld instrument according to one of the previous claims, **characterized in that** the section C is formed as an arc or loop.

5. Electrode (16) for an electrosurgical handheld instrument according to one of the previous claims, **characterized in that** the wire (18) consists of an electrically conductive material with a very high melting point, such as preferably tungsten, molybdenum or tantalum.

6. Electrode (16) for an electrosurgical handheld instrument according to claim 5, **characterized in that** the melting point is at least 2000°C, preferably more than 3000°C, in particular more than 3400°C.

7. Electrode (16) for an electrosurgical handheld instrument according to one of the previous claims, **characterized in that** the wire (18) has a round or circular cross-section (25).

8. Electrode (16) for an electrosurgical handheld instrument according to one of the previous claims, **characterized in that** the sections R, L and C lie in one plane or that the sections R, L and C lie in two different planes.

9. Electrode (16) for an electrosurgical handheld instrument according to one of the previous claims, **characterized in that** the sections R, L and C are arranged at right angles to each other.

10. Electrode (16) for an electrosurgical handheld instrument according to one of the previous claims, **characterized in that** the wire (18) has a cross-section (25) of 0.8 mm.
